# EUROPÄISCHE PATENTSCHRIFT

(11) **EP 3 092 970 B1**
(45) Veröffentlichungstag und Bekanntmachung des Hinweises auf die Patenterteilung: **30.12.2020**
(21) Anmeldenummer: 16167146.6
(22) Anmeldetag: 26.04.2016
(51) Int. Cl.: H05B 47/105, A61B 90/30

(54) **VERFAHREN UND VORRICHTUNG ZUR ANSTEUERUNG EINER OPERATIONSLEUCHTE**
METHOD AND DEVICE FOR CONTROLLING AN OPERATING LIGHT
PROCÉDÉ ET DISPOSITIF DE COMMANDE D'UNE LAMPE CHIRURGICALE

(30) Priorität: 28.04.2015 DE 102015106519
(43) Veröffentlichungstag der Anmeldung: 16.11.2016
(73) Patentinhaber: Berchtold GmbH & Co. KG, 78532 Tuttlingen (DE)
(72) Erfinder: Jacobi, Leif, 78532 Tuttlingen (DE)
(74) Vertreter: V.O.

(56) Entgegenhaltungen:
- EP-A2- 1 342 947
- WO-A1-2014/016104
- DE-A1-102008 019 191
- DE-A1-102008 039 791
- DE-A1-102009 037 316
- DE-A1-102011 007 201

## Beschreibung

Die vorliegende Erfindung betrifft ein Verfahren und eine Vorrichtung zur Ansteuerung einer ein Operationsfeld beleuchtenden Operationsleuchte.

Bei der Beleuchtung eines Operationsfelds kommt der Einstellung der richtigen Helligkeit große Bedeutung zu. Hierzu ist es aus dem Stand der Technik bekannt, den Abstand zwischen Operationsleuchte und Operationsfeld zu messen und die Beleuchtungsstärke abhängig vom Abstand konstant zu halten (vgl. EP 1 342 947 A2). Auch ist es bekannt, eine Leuchtdichtemessung vorzunehmen und die Helligkeit der Operationsleuchte entsprechend anzupassen. Eine Konstanthaltung der Leuchtfeldgröße durch Fokussieren in Abhängigkeit von einem gemessenen Abstand ist aus dem Stand der Technik ebenfalls bekannt.

Aus der DE 10 2009 037316 A1 ist ein Verfahren zur Ansteuerung einer ein Operationsfeld beleuchtenden Operationsleuchte bekannt, bei dem die Ränder des Operationsfelds erkannt werden und ein Steuersignal in Abhängigkeit von der Ausdehnung des Operationsfelds erzeugt wird. Durch Gestensteuerung kann unter anderem die Gestalt des beleuchteten Bereichs verändert werden.

Bei der Verwendung bekannter Operationsleuchten kann es bei falsch eingestellter Leuchtfeldgröße zu einer übermäßigen Beleuchtung des durch Tücher bzw. andere Abdeckungen abgedeckten Bereiches um das eigentliche Operationsfeld herum kommen. Hierdurch wird ein helles Umfeld geschaffen, auf welches der Operateur adaptiert, wodurch das eigentliche Operationsfeld noch dunkler erscheint. Darüber hinaus können unzulässig hohe Leuchtdichteunterschiede im Gesichtsfeld des Operateurs zu negativen physiologischen und psychologischen Wirkungen führen, welche den Sehkomfort und die Sehleistung beeinträchtigen können.

Es ist die Aufgabe der vorliegenden Erfindung, ein Verfahren und eine Vorrichtung zur Ansteuerung einer ein Operationsfeld beleuchtenden Operationsleuchte zu schaffen, mit denen unabhängig von der Entfernung der Operationsleuchte vom Operationsfeld eine optimierte Einstellung der Lichtintensität erfolgen kann.

Die Lösung dieser Aufgabe erfolgt durch die Merkmale der unabhängigen Patentansprüche.

Das erfindungsgemäße Verfahren umfasst folgende Schritte in der genannten Reihenfolge: Zunächst wird eine ortsselektive Ermittlung der Leuchtdichteverteilung in einem Messfeld durchgeführt, das größer als eine maximale Leuchtfeldgröße der Operationsleuchte ist. Die maximale Leuchtfeldgröße der Operationsleuchte kann beispielsweise durch Fokussieren oder durch Ansteuern einzelner Leuchtmittel innerhalb der Operationsleuchte verändert werden, d.h. die maximale Leuchtfeldgröße der Operationsleuchte ist bekannt. Entsprechend kann ein Messfeld gewählt werden, das größer als die maximale Leuchtfeldgröße ist und innerhalb dieses Messfeldes kann ortsselektiv, d.h. ortsaufgelöst die Leuchtdichteverteilung ermittelt werden.

Aus der ermittelten Leuchtdichteverteilung lässt sich auf die tatsächliche Größe des Operationsfelds rückschließen, so dass in einem zweiten Schritt die Leuchtfeldgröße der Operationsleuchte an die Größe des im ersten Schritt ermittelten Operationsfelds unter Berücksichtigung der Leuchtdichteverteilung angepasst werden kann. Die Leuchtfeldgröße kann also in dem zweiten Schritt so vergrößert oder verkleinert werden, dass das erzeugte Leuchtfeld an die Größe des Operationsfelds angepasst ist, d.h. im Wesentlichen der Größe des Operationsfelds entspricht.

Anschließend kann in einem dritten Schritt die Lichtintensität der Operationsleuchte unter Berücksichtigung der Leuchtdichte der Umgebung des Operationsfelds innerhalb des Messfeldes eingestellt werden. Die Lichtintensität der Operationsleuchte kann so angepasst werden, dass das Leuchtdichteverhältnis zwischen Operationsfeld und Umgebung des Operationsfelds optimiert ist. Ist beispielsweise die Umgebung des Operationsfelds sehr schwach reflektierend, so kann die Lichtintensität der Operationsleuchte höher eingestellt werden. Ist die Umgebung stark reflektierend, so kann die Lichtintensität geringer eingestellt werden, um Blendeffekte zu vermeiden. Es erfolgt also eine Einstellung der Lichtintensität der Operationsleuchte unter Berücksichtigung der Leuchtdichte der innerhalb des Messfelds liegenden Umgebung des Operationsfelds.

Erfindungsgemäß wird zur Einstellung der angepassten Lichtintensität ein Verhältnis zwischen der Leuchtdichte im Operationsfeld und der Leuchtdichte in der Umgebung des Operationsfelds vorgegeben, wobei das vorgegebene Verhältnis insbesondere mit der Größe des Leuchtfelds variieren kann. So kann beispielsweise vorgegeben werden, dass die Leuchtdichte innerhalb des Operationsfelds stets um einen bestimmten Prozentsatz höher ist als die Leuchtdichte in der Umgebung des Operationsfelds. Auch kann beispielsweise vorgegeben werden, dass die Leuchtdichte bei kleinerem Operationsfeld geringer und bei größerem Operationsfeld höher ist oder dass die Leuchtdichte bei kleinerem Operationsfeld höher und bei größerem Operationsfeld geringer ist.

Vorteilhafte Ausführungsformen der Erfindung sind in der Beschreibung, der Zeichnung sowie den Unteransprüchen beschrieben.

Nach einer ersten vorteilhaften Ausführungsform kann aus der ermittelten Leuchtdichteverteilung ein Leuchtdichteobjekt ermittelt werden, das dem Operationsfeld entspricht. Durch die ortsselektive Messung der Leuchtdichte lässt sich nämlich mittels bekannter Verfahren, beispielsweise dem Gradientenverfahren, ein Leuchtdichteobjekt definieren, dessen Außenkontur der Außenkontur des Operationsfelds entspricht. Das so ermittelte Leuchtdichteobjekt kann dann weiter verwendet werden, um die Anpassung der Leuchtfeldgröße der Operationsleuchte vorzunehmen.

Die Einstellung einer gewünschten und an die Umgebung des Operationsfelds angepassten Lichtintensität kann auf verschiedene Art und Weise erfolgen. In jedem Fall wird die Lichtintensität so angepasst, dass diese für den Operateur in Abhängigkeit von der Helligkeit der Umgebung des Operationsfelds optimiert ist. Nach einer vorteilhaften Ausführungsform kann zur Einstellung der anpassten Lichtintensität ein Kennlinienfeld herangezogen werden, in dem in Abhängigkeit von der Größe des Operationsfelds und von der Helligkeit der Umgebung des Operationsfelds Lichtintensitäten abgespeichert sind. Da die Helligkeit in der Umgebung des Operationsfelds und auch die Lichtfeldgröße bekannt ist, kann aus dem Kennlinienfeld ein optimierter Helligkeitswert ausgelesen werden, der beispielsweise empirisch ermittelt worden ist.

Nach einer weiteren vorteilhaften Ausführungsform kann aus einem ermittelten Leuchtdichteobjekt eine Leuchtfeldgröße errechnet werden, die das Leuchtdichteobjekt einbeschreibt. Mit anderen Worten kann der geometrische Mittelpunkt des Leuchtdichteobjekts und dessen Außenkontur ermittelt werden und aus diesen Daten kann eine Leuchtfeldgröße erreicht werden, die das zuvor ermittelte Leuchtdichteobjekt, d.h. die Operationsstelle, vollständig einbeschreibt.

Nach einer weiteren vorteilhaften Ausführungsform kann die ortsselektive Ermittlung der Leuchtdichteverteilung als punktuelle Leuchtdichtemessung mittels mehrerer Einzelsensoren erfolgen. Alternativ ist es möglich, die Leuchtdichteverteilung mit einer kalibrierten Kamera, beispielsweise einer Graustufenkamera zu ermitteln, was auf kostengünstige Weise möglich ist. Schließlich ist auch die Verwendung einer Leuchtdichtekamera möglich.

Nach einer weiteren vorteilhaften Ausführungsform kann zur besseren Erkennung des Operationsfeldrandes zumindest eine Reflexionsmarke verwendet werden. Eine solche Reflexionsmarke kann beispielsweise nur temporär und manuell vor der eigentlichen Operation eingesetzt werden. Nach einer weiteren vorteilhaften Ausführungsform kann eine Operationsfeldabdeckung verwendet werden, deren Rand mit einer im nicht sichtbaren Bereich (beispielsweise im Infrarotbereich oder im nahen UV-Bereich) oder aber im sichtbaren Bereich reflektierenden Reflexionsmarke versehen ist. Eine solche Operationsfeldabdeckung, wie sie üblicherweise verwendet wird, weist eine Öffnung mit einem umlaufenden Rand auf, so dass der umlaufende Rand mit einer durchgehenden oder mehreren einzelnen Reflexionsmarken versehen werden kann, was die automatisierte Erfassung der Größe bzw. Kontur des Operationsfelds erleichtert.

Die Anpassung der Leuchtfeldgröße an die Größe des Operationsfelds kann grundsätzlich so erfolgen, dass die Größe des Leuchtfelds genauso groß ist wie die Erstreckung des Operationsfelds. Nach einer weiteren Ausführungsform kann jedoch auch die Leuchtfeldgröße etwas größer als die Größe des Operationsfelds eingestellt werden, so dass das Leuchtfeld auf jeden Fall die Außenkontur des Operationsfelds überdeckt.

Nach einem weiteren Aspekt der vorliegenden Erfindung betrifft diese eine Vorrichtung zur Durchführung eines Verfahrens der vorstehend beschriebenen Art, wobei die Vorrichtung eine Sensorik zur ortsselektiven Ermittlung der Leuchtdichteverteilung in einem Messfeld umfasst. Weiterhin ist eine Einrichtung zur Anpassung der Leuchtfeldgröße einer Operationsleuchte vorgesehen und eine Einrichtung zur Einstellung der Lichtintensität der Operationsleuchte ist so ausgebildet, dass die Lichtintensität an die Umgebung eines Operationsfelds innerhalb des Messfelds angepasst werden kann. Bevorzugt ist diese Vorrichtung in einen Handgriff für eine Operationsleuchte integriert, so dass bereits bestehende Operationsleuchten mit der Vorrichtung nachgerüstet werden können. Alternativ kann die Vorrichtung in einem Leuchtenkörper der Operationsleuchte oder in Handgriff und Leuchtenkörper integriert sein.

Nachfolgend wird die vorliegende Erfindung rein beispielhaft anhand einer vorteilhaften Ausführungsform und unter Bezugnahme auf die beigefügten Zeichnungen beschrieben. Es zeigen:
- Fig. 1: eine perspektivische Ansicht eines von einer Operationsleuchte beleuchteten Operationsfelds;
- Fig. 2 und Fig. 3: die Anpassung der Leuchtfeldgröße an das Operationsfeld; und
- Fig. 4: ein Regelungsschema für die Einstellung der Leuchtfeldgröße und die Regelung der Leuchtdichte innerhalb des Operationsfelds.

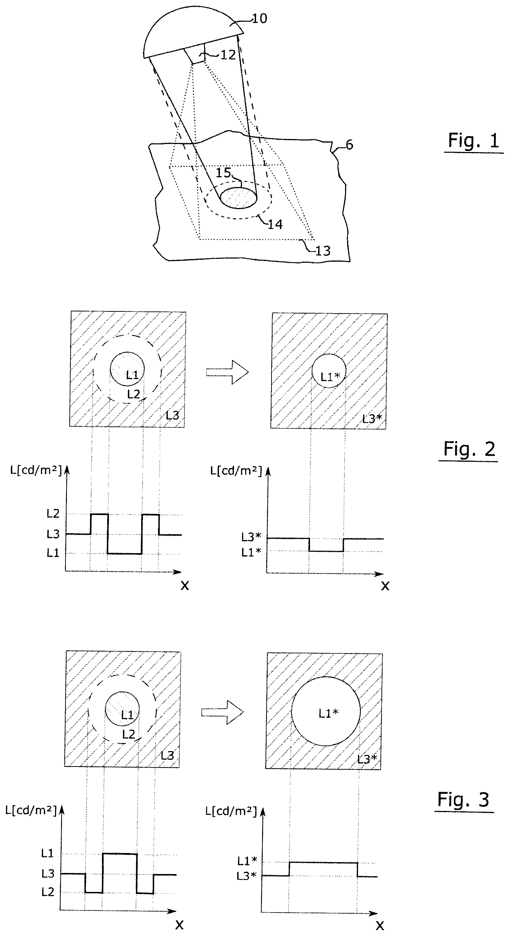

Fig. 1 zeigt eine perspektivische Ansicht einer nur schematisch dargestellten Operationsleuchte 10, in deren zentralem Handgriff 12 Messmittel sowie Elektronik zur Messmittelsteuerung, Informationsverarbeitung und Generierung von Steuersignalen für die Leuchte sowie zur Überwachung von Regelgrößen vorgesehen sind.
Die Integration sowohl der Messmittel (Sensorik in Form einer Kamera oder in Form von Lichtsensoren) wie auch der Informationsverarbeitung in Form von Hard- und Software im Handgriff ermöglicht ein kundenorientiertes Nachrüsten der erfindungsgemäßen Funktionen. Die in den Handgriff 12 integrierte Einheit überträgt lediglich Steuersignale an den Leuchtenkörper, ähnlich einer Leuchtenbedienung, um die Lichtintensität und die Leuchtfeldgröße der Operationsleuchte zu verändern.

Wie Fig. 1 verdeutlicht, beleuchtet die Operationsleuchte 10 ein Operationsfeld 15 mit einer vorgewählten Leuchtfeldgröße 14, die gestrichelt dargestellt und größer als das eigentliche Operationsfeld 15 ist, so dass auch die Umgebung des Operationsfelds 15 in einem Ringstreifen beleuchtet wird.

Eine innerhalb des Handgriffs 12 vorgesehene Sensorik, vorzugsweise eine Leuchtdichtekamera, dient zur Erfassung von Helligkeitsunterschieden an verschiedenen Positionen im Arbeitsbereich des Operateurs und die erfassten Helligkeitsunterschiede können zur Position der Leuchte bzw. zumindest zur optischen Achse der Leuchte korreliert werden, so dass die Leuchtdichteverteilung in einem Messfeld 13 ortsselektiv ermittelt werden kann, das größer als die maximale Leuchtfeldgröße 14 der Operationsleuchte ist.

Gemäß dem erfindungsgemäßen Verfahren wird anschließend zunächst die Leuchtfeldgröße der Operationsleuchte an die Größe des Operationsfelds 15 unter Berücksichtigung der ermittelten Leuchtdichteverteilung angepasst.

Fig. 2 verdeutlicht die Anpassung der Leuchtfeldgröße bei zu großem Anfangsleuchtfeld. Hierbei erfolgt zunächst eine erste Aufnahme der Leuchtdichte im Kamerabildfeld, wobei die Leuchte 10 auf das Operationsfeld ausgerichtet ist (Fig. 2, Bezugszeichen A). Anschließend können die Bereichsgrenzen bzw. die Leuchtdichteobjekte im Bildfeld berechnet werden, so dass die mittleren Leuchtdichten in den aufgefundenen Bereichen L1 (Operationsfeld), L2 (beleuchteter Bereich) und L3 (Umgebung) berechnet werden können. Aufgrund der so ermittelten Daten lässt sich dann die Leuchtfeldgröße, d.h. die räumliche Ausdehnung des Bereichs mit der Leuchtdichte L2 so verkleinern, dass der beleuchtete Bereich im Wesentlichen dem Operationsfeld entspricht.

Im Anschluss kann die Beleuchtungsstärke so variiert werden (Fig. 2, Bezugszeichen B), dass die Leuchtdichte L1* im Operationsfeld einen für die Sehaufgabe und die vorliegende Umfeldleuchtdichte L3* optimierten Wert annimmt, wobei sich die Umfeldleuchtdichte L3* durch die Anpassung der Leuchtfeldgröße ebenfalls in einem gewissen Bereich im Vergleich zu L3 vor der Größenverstellung verändern kann.

Die in Fig. 2 dargestellten Leuchtdichtewerte sind rein beispielhaft angegeben. So ist es auch möglich, dass die Leuchtdichte L1* größer ist als die Leuchtdichte L3*.

Fig. 3 verdeutlicht eine Situation, bei der das anfängliche Leuchtfeld L1 kleiner als das Operationsfeld L2 ist. Auch hier wird die Leuchtfeldgröße verändert, so dass diese im Wesentlichen dem Operationsfeld entspricht und anschließend wird die Leuchtdichte auf einen Wert L1* eingestellt, der gegenüber der Umgebungsleuchtdichte L3* optimiert ist. In allen Fällen wird das erfindungsgemäße Verfahren ohne eine Abstandsmessung durchgeführt.

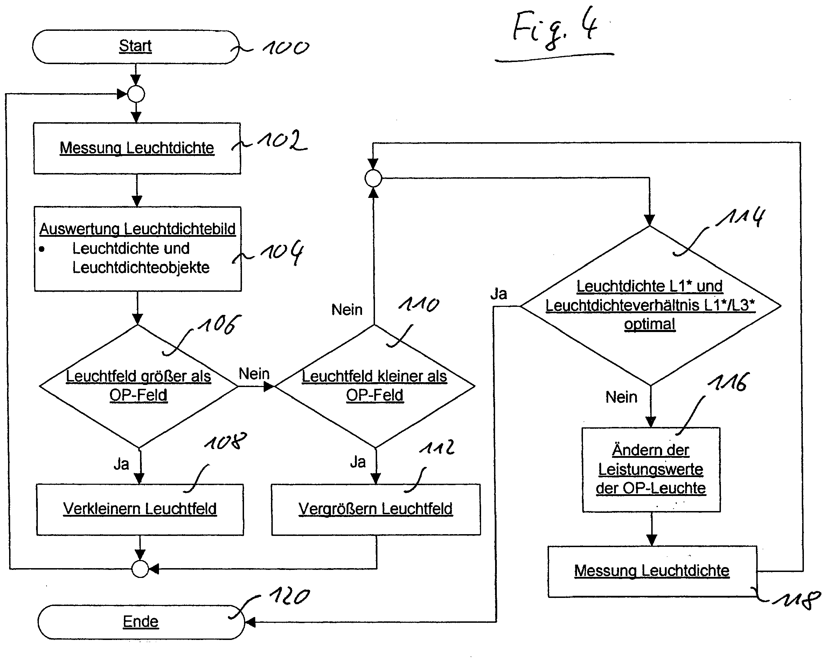

Das vorstehende Verfahren, das als Steuerung oder als Regelung ausgebildet sein kann, wird nachfolgend nochmals anhand von Fig. 4 erläutert:
Das Verfahren beginnt mit dem Schritt 100 und führt anschließend im Schritt 102 eine Messung der Leuchtdichte durch, wobei im Schritt 104 eine Auswertung des ermittelten Leuchtdichtebildes erfolgt und aus der Leuchtdichteverteilung eines oder mehrere Leuchtdichteobjekte ermittelt werden. Im nachfolgenden Schritt 106 wird abgefragt, ob das Leuchtfeld 14 der Operationsleuchte 10 größer als das durch die ermittelte Leuchtdichteverteilung festgestellte Operationsfeld 15 ist. Ist dies der Fall, so wird im nachfolgenden Schritt 108 das Leuchtfeld verkleinert und es wird zum Schritt 102 zurückgekehrt. Ist dies nicht der Fall, wird im Schritt 110 abgefragt, ob das Leuchtfeld kleiner als das Operationsfeld 15 ist. Ist dies der Fall, so wird im Schritt 112 das Leuchtfeld vergrößert und zum Schritt 102 zurückgekehrt. Ist dies nicht der Fall, so besitzt das Leuchtfeld die Größe des Operationsfelds und es kann anschließend die Lichtintensität der Operationsleuchte unter Berücksichtigung der Umgebung des Operationsfelds optimiert werden. Hierzu wird im Schritt 114 abgefragt, ob die Leuchtdichte L1* des jetzt vorhandenen Leuchtfelds in einem optimierten Leuchtdichteverhältnis zur Leuchtdichte der Umgebung L3* steht. Ist dies der Fall, so kann das Verfahren im Schritt 120 beendet werden. Ist dies nicht der Fall, wird im Schritt 116 die Lichtintensität der Operationsleuchte durch Ändern deren Leistungswerte, d.h. durch ein Regeln der Intensität geändert. Anschließend wird im Schritt 118 erneut die Leuchtdichte gemessen und es wird zum Schritt 114 zurückgekehrt.

Es versteht sich, dass anstelle des Schritts 120 auch zum Schritt 102 oder zum Schritt 114 zurückgekehrt werden kann, um eine kontinuierliche Regelung zu erzielen. Auch ist offensichtlich, dass sich die vorstehend beschriebene Erfindung nicht nur auf Operationsleuchten sondern in gleicher Weise auf beliebige andere Leuchten, wie Untersuchungsleuchten und dgl. anwenden lässt.

Zur Aktivierung des vorstehend beschriebenen Verfahrens kann an einer Steuerung der Operationsleuchte ein eigenes Schaltelement vorgesehen werden. Außerdem ist es möglich, jederzeit die automatische Intensitätsanpassung manuell abzuschalten. Außerdem kann vorgesehen werden, die Helligkeit der Leuchte und/oder die Leuchtfeldgröße zu reduzieren, wenn beispielsweise aufgrund der Leuchtdichtemessung festgestellt wird, dass die Leuchte nicht in Richtung des Operationsfelds sondern in den Raum orientiert ist. Hierdurch wird die Blendung von ärztlichem Personal verhindert.

## Patentansprüche

1. Verfahren zur Ansteuerung einer ein Operationsfeld beleuchtenden Operationsleuchte (10), umfassend die folgenden Schritte in der genannten Reihenfolge:
a) ortsselektive Ermittlung der Leuchtdichteverteilung in einem Messfeld (13), das größer als eine maximale Leuchtfeldgröße (14) der Operationsleuchte (10) ist,
b) Anpassung der Leuchtfeldgröße der Operationsleuchte (10) an die Größe des Operationsfelds (15) unter Berücksichtigung der Leuchtdichteverteilung, und
c) Einstellung einer Lichtintensität der Operationsleuchte unter Berücksichtigung der Leuchtdichte der innerhalb des Messfelds (13) liegenden Umgebung des Operationsfelds,
**gekennzeichnet dadurch, dass**
im Schritt c) zur Einstellung der Lichtintensität ein Verhältnis zwischen der Leuchtdichte im Operationsfeld und der Leuchtdichte in der Umgebung des Operationsfelds vorgegeben wird.

2. Verfahren nach Anspruch 1,
**dadurch gekennzeichnet, dass**
aus der Leuchtdichteverteilung ein Leuchtdichteobjekt ermittelt wird, das dem Operationsfeld (15) entspricht.

3. Verfahren nach Anspruch 1 oder 2,
**dadurch gekennzeichnet, dass** im Schritt c) zur Einstellung der Lichtintensität ein Kennlinienfeld verwendet wird, in dem in Abhängigkeit von der Größe des Operationsfelds (15) und von der Helligkeit der Umgebung des Operationsfelds Lichtintensitäten abgespeichert sind.

4. Verfahren nach Anspruch 1, 2 oder 3,
**dadurch gekennzeichnet, dass** das vorgegebene Verhältnis mit der Leuchtfeldgröße variiert.

5. Verfahren nach Anspruch 2,
**dadurch gekennzeichnet, dass** aus dem Leuchtdichteobjekt eine insbesondere kreisförmige Leuchtfeldgröße errechnet wird, die das Leuchtdichteobjekt einbeschreibt.

6. Verfahren nach zumindest einem der vorstehenden Ansprüche,
**dadurch gekennzeichnet, dass** die ortsselektive Ermittlung der Leuchtdichteverteilung als punktuelle Leuchtdichtemessung mittels mehrerer Einzelsensoren erfolgt.

7. Verfahren nach zumindest einem der vorstehenden Ansprüche 1 - 5,
**dadurch gekennzeichnet, dass** die Leuchtdichteverteilung mit einer kalibrierten Graustufenkamera ermittelt wird.

8. Verfahren nach zumindest einem der vorstehenden Ansprüche,
**dadurch gekennzeichnet, dass** zur besseren Erkennung des Operationsfeldrandes zumindest eine Reflexionsmarke verwendet wird.

9. Verfahren nach Anspruch 8,
**dadurch gekennzeichnet, dass**
zur besseren Erkennung des Operationsfeldrandes eine Operationsfeldabdeckung verwendet wird, deren Rand mit einer im nicht sichtbaren oder im sichtbaren Bereich reflektierenden Reflexionsmarke versehen ist.

10. Verfahren nach zumindest einem der vorstehenden Ansprüche,
**dadurch gekennzeichnet, dass**
im Schritt b) die Leuchtfeldgröße etwas größer als die Größe des Operationsfelds eingestellt wird.

11. Vorrichtung zur Durchführung eines Verfahrens nach zumindest einem der vorstehenden Ansprüche, umfassend
eine Sensorik zur ortsselektiven Ermittlung der Leuchtdichteverteilung in einem Messfeld,
eine Einrichtung zur Anpassung der Leuchtfeldgröße einer Operationsleuchte, **gekennzeichnet durch** eine Einrichtung zur Einstellung der Lichtintensität der Operationsleuchte derart, dass die Leuchtdichte der innerhalb des Messfelds (13) liegenden Umgebung des Operationsfelds berücksichtigt wird, wobei ein Verhältnis zwischen der Leuchtdichte im Operationsfeld und der Leuchtdichte in der Umgebung des Operationsfelds vorgegeben wird.

12. Vorrichtung nach Anspruch 11,
**dadurch gekennzeichnet, dass**
diese innerhalb eines Handgriffes für eine Operationsleuchte unterbringbar ist.

## Claims

1. Method for controlling an operating light (10) illuminating an operating field, comprising the following steps in the order mentioned:
a) location-selective determination of the luminance distribution in a measuring field (13) that is larger than a maximum luminous field size (14) of the operating light (10),
b) adaptation of the luminous field size of the operating light (10) to the size of the operating field (15) taking into account the luminance distribution,
and
c) setting a light intensity of the operating light taking into account the luminance of the surroundings of the operating field within the measuring field (13),
**characterised in that**
in step c) a ratio between the luminance in the operating field and the luminance in the surroundings of the operating field is specified for setting the light intensity.

2. Method according to claim 1,
**characterised in that**
a luminance object, which corresponds to the operating field (15), is determined from the luminance distribution.

3. Method according to claim 1 or 2,
**characterised in that**
in step c) a family of characteristics is used to set the light intensity, in which light intensities are stored as a function of the size of the operating field (15) and the brightness of the surroundings of the operating field.

4. Method according to claim 1, 2 or 3,
**characterised in that**
the specified ratio varies with the luminous field size.

5. Method according to claim 2,
**characterised in that**
an in particular circular luminous field size, which outlines the luminance object,
is calculated from the luminance object.

6. Method according to at least one of the preceding claims,
**characterised in that**
the location-selective determination of the luminance distribution takes place as a punctiform luminance measurement by means of several individual sensors.

7. Method according to at least one of the preceding claims 1-5,
**characterised in that**
the luminance distribution is determined with a calibrated greyscale camera.

8. Method according to at least one of the preceding claims,
**characterised in that**
at least one reflection mark is used to better identify the edge of the operating field.

9. Method according to claim 8,
**characterised in that**
for better recognition of the edge of the operating field, an operating field cover is used, the edge of which is provided with a reflection mark that is reflective in the non-visible or in the visible range.

10. Method according to at least one of the preceding claims,
**characterised in that**
in step b) the luminous field size is set somewhat larger than the size of the operating field.

11. Device for performing a method according to at least one of the preceding claims, comprising
a sensor system for the location-selective determination of the luminance distribution in a measuring field,
an apparatus for adapting the luminous field size of an operating light,
**characterised by**
an apparatus for setting the light intensity of the operating light in such a way that the luminance of the surroundings of the operating field within the measuring field (13) is taken into account, wherein a ratio between the luminance in the operating field and the luminance in the surroundings of the operating field is specified.

12. Device according to claim 11,
**characterised in that**
this device can be accommodated within a handle for an operating light.

## Revendications

1. Procédé de pilotage d'une lampe chirurgicale (10) éclairant un champ opératoire, comprenant les étapes suivantes dans l'ordre mentionné :
(a) la détermination localement sélective de la répartition de densité lumineuse dans un champ de mesure (13), qui est plus grand qu'une taille de champ lumineux (14) maximale de la lampe chirurgicale (10) ;
(b) l'adaptation de la taille de champ lumineux de la lampe chirurgicale (10) à la taille du champ opératoire (15) en tenant compte de la répartition de densité lumineuse ; et
(c) le réglage de l'intensité lumineuse de la lampe chirurgicale en tenant compte de la densité lumineuse de l'environnement, situé à l'intérieur du champ de mesure (13), du champ opératoire,
**caractérisé en ce que**
lors de l'étape c) de réglage de l'intensité lumineuse, un rapport entre la densité lumineuse dans le champ opératoire et la densité lumineuse dans l'environnement du champ opératoire est spécifié.

2. Procédé selon la revendication 1,
**caractérisé en ce que**
est déterminé à partir de la répartition de densité lumineuse un objet de densité lumineuse, qui correspond au champ opératoire (15).

3. Procédé selon la revendication 1 ou 2,
**caractérisé en ce que**
lors de l'étape c) de réglage de l'intensité lumineuse, un champ de caractéristiques est utilisé, dans lequel des intensités lumineuses sont sauvegardées en fonction de la taille du champ opératoire (15) et de la clarté de l'environnement du champ opératoire.

4. Procédé selon la revendication 1, 2 ou 3,
**caractérisé en ce que**
le rapport spécifié varie avec la taille de champ lumineux.

5. Procédé selon la revendication 2,
**caractérisé en ce que**
est calculée à partir de l'objet de densité lumineuse une taille de champ lumineux en particulier de forme circulaire, qui inscrit l'objet de densité lumineuse.

6. Procédé selon au moins l'une quelconque des revendications précédentes,
**caractérisé en ce que**
la détermination localement sélective de la répartition de densité lumineuse et effectuée en tant que mesure de densité lumineuse ponctuelle au moyen de plusieurs capteurs individuels.

7. Procédé selon au moins l'une quelconque des revendications précédentes 1 - 5,
**caractérisé en ce que**
la répartition de densité lumineuse est déterminée avec une caméra à valeurs de gris étalonnée.

8. Procédé selon au moins l'une quelconque des revendications précédentes,
**caractérisé en ce que**
au moins un repère de réflexion est utilisé pour mieux identifier le bord de champ opératoire.

9. Procédé selon la revendication 8,
**caractérisé en ce que**
est utilisé, pour mieux identifier le bord de champ opératoire, un recouvrement de champ opératoire, dont le bord est pourvu d'un repère de réflexion à réflexion dans le domaine non visible ou dans le domaine visible.

10. Procédé selon au moins l'une quelconque des revendications précédentes,
**caractérisé en ce que**
lors de l'étape b), la taille de champ lumineux est réglée à peu près plus grande que la taille du champ opératoire.

11. Dispositif de mise en œuvre d'un procédé selon au moins l'une quelconque des revendications précédentes, comprenant
un mécanisme de détection pour la détermination localement sélective de la répartition de densité lumineuse dans un champ de mesure,
un système d'adaptation de la taille de champ lumineux d'une lampe chirurgicale, **caractérisé par**
un système de réglage de l'intensité lumineuse de la lampe chirurgicale de telle manière que la densité lumineuse de l'environnement, situé à l'intérieur du champ de mesure (13), du champ opératoire est prise en compte, dans lequel un rapport entre la densité lumineuse dans le champ opératoire et la densité lumineuse dans l'environnement du champ opératoire est spécifié.

12. Dispositif selon la revendication 11,
**caractérisé en ce que** celui-ci peut être abrité à l'intérieur d'une poignée pour une lampe chirurgicale.
